(19) **Europäisches Patentamt / European Patent Office / Office européen des brevets**

(11) **EP 4 067 372 A1**

(12) # EUROPEAN PATENT APPLICATION
published in accordance with Art. 153(4) EPC

(43) Date of publication:
**05.10.2022 Bulletin 2022/40**

(21) Application number: **20893352.3**

(22) Date of filing: **23.10.2020**

(51) International Patent Classification (IPC):
$C07K\ 14/47$ (2006.01)  $C12N\ 15/12$ (2006.01)
$C12N\ 15/31$ (2006.01)  $G01N\ 33/53$ (2006.01)
$G01N\ 33/543$ (2006.01)  $G01N\ 33/96$ (2006.01)

(52) Cooperative Patent Classification (CPC):
**C07K 14/195; C07K 14/435; C07K 14/47;
G01N 33/53; G01N 33/543; G01N 33/96**

(86) International application number:
**PCT/JP2020/039893**

(87) International publication number:
**WO 2021/106453 (03.06.2021 Gazette 2021/22)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **29.11.2019 JP 2019216784
21.08.2020 JP 2020140434**

(71) Applicant: **TOYOBO CO., LTD.**
**Osaka-shi**
**Osaka 5300001 (JP)**

(72) Inventors:
• **MISHIMA, Ayaka**
  **Tsuruga-shi, Fukui 914-8550 (JP)**
• **SUMIDA, Yosuke**
  **Osaka-shi, Osaka 530-8230 (JP)**

(74) Representative: **Marks & Clerk LLP**
**15 Fetter Lane**
**London EC4A 1BW (GB)**

(54) **RECOMBINANT C-REACTIVE PROTEIN**

(57) The accuracy of immunoassay using a latex reagent is improved in a high CRP concentration range. Provided are C-reactive proteins generated by genetic recombination, 55% or more of the C-reactive proteins having a pyro-glutamylated N-terminal.

EP 4 067 372 A1

SEQUENCE LISTING

<110>  TOYOBO CO., LTD

<120>  Recombinant C-reactive Protein

<130>  200307WO01

<141>  2020-08-21

<160>  10

<170>  PatentIn version 3.1

<210>  1
<211>  206
<212>  PRT
<213>  Homo sapiens
<400>  1

```
Gln Thr Asp Met Ser Arg Lys Ala Phe Val Phe Pro Lys Glu Ser Asp
1               5                   10                  15
Thr Ser Tyr Val Ser Leu Lys Ala Pro Leu Thr Lys Pro Leu Lys Ala
            20                  25                  30
Phe Thr Val Cys Leu His Phe Tyr Thr Glu Leu Ser Ser Thr Arg Gly
            35                  40                  45
Thr Val Phe Ser Arg Met Pro Pro Arg Asp Lys Thr Met Arg Phe Phe
        50                  55                  60
Ile Phe Trp Ser Lys Asp Ile Gly Tyr Ser Phe Thr Val Gly Gly Ser
65                  70                  75                  80
Glu Ile Leu Phe Glu Val Pro Glu Val Thr Val Ala Pro Val His Ile
                85                  90                  95
Cys Thr Ser Trp Glu Ser Ala Ser Gly Ile Val Glu Phe Trp Val Asp
            100                 105                 110
Gly Lys Pro Arg Val Arg Lys Ser Leu Lys Lys Gly Tyr Thr Val Gly
            115                 120                 125
Ala Glu Ala Ser Ile Ile Leu Gly Gln Glu Gln Asp Ser Phe Gly Gly
            130                 135                 140
Asn Phe Glu Gly Ser Gln Ser Leu Val Gly Asp Ile Gly Asn Val Asn
145                 150                 155                 160
Met Trp Asp Phe Val Leu Ser Pro Asp Glu Ile Asn Thr Ile Tyr Leu
                165                 170                 175
Gly Gly Pro Phe Ser Pro Asn Val Leu Asn Trp Arg Ala Leu Lys Tyr
            180                 185                 190
Glu Val Gln Gly Glu Val Phe Thr Lys Pro Gln Leu Trp Pro
            195                 200                 205
```

<210>  2
<211>  206
<212>  PRT
<213>  Homo sapiens
<400>  2

```
Gln Thr Asp Met Ser Arg Lys Ala Phe Val Phe Pro Lys Glu Ser Asp
1               5                   10                  15
Thr Ser Tyr Val Ser Leu Lys Ala Pro Leu Thr Lys Pro Leu Lys Ala
            20                  25                  30
Phe Thr Val Cys Leu His Phe Tyr Thr Glu Leu Ser Ser Thr Arg Gly
            35                  40                  45
Tyr Ser Ile Phe Ser Tyr Ala Thr Lys Arg Gln Asp Asn Glu Ile Leu
        50                  55                  60
Ile Phe Trp Ser Lys Asp Ile Gly Tyr Ser Phe Thr Val Gly Gly Ser
65                  70                  75                  80
```

14

```
Glu Ile Leu Phe Glu Val Pro Glu Val Thr Val Ala Pro Val His Ile
                85              90              95
Cys Thr Ser Trp Glu Ser Ala Ser Gly Ile Val Glu Phe Trp Val Asp
            100             105             110
Gly Lys Pro Arg Val Arg Lys Ser Leu Lys Lys Gly Tyr Thr Val Gly
        115             120             125
Ala Glu Ala Ser Ile Ile Leu Gly Gln Glu Gln Asp Ser Phe Gly Gly
    130             135             140
Asn Phe Glu Gly Ser Gln Ser Leu Val Gly Asp Ile Gly Asn Val Asn
145             150             155             160
Met Trp Asp Phe Val Leu Ser Pro Asp Glu Ile Asn Thr Ile Tyr Leu
            165             170             175
Gly Gly Pro Phe Ser Pro Asn Val Leu Asn Trp Arg Ala Leu Lys Tyr
        180             185             190
Glu Val Gln Gly Glu Val Phe Thr Lys Pro Gln Leu Trp Pro
    195             200             205
```

```
<210>  3
<211>  227
<212>  PRT
<213>  Artificial Sequence
<400>  3
Met Lys Gln Ser Thr Ile Ala Leu Ala Leu Leu Pro Leu Leu Phe Thr
1               5               10              15
Pro Val Thr Lys Ala Gln Thr Asp Met Ser Arg Lys Ala Phe Val Phe
            20              25              30
Pro Lys Glu Ser Asp Thr Ser Tyr Val Ser Leu Lys Ala Pro Leu Thr
        35              40              45
Lys Pro Leu Lys Ala Phe Thr Val Cys Leu His Phe Tyr Thr Glu Leu
        50              55              60
Ser Ser Thr Arg Gly Thr Val Phe Ser Arg Met Pro Pro Arg Asp Lys
65              70              75              80
Thr Met Arg Phe Phe Ile Phe Trp Ser Lys Asp Ile Gly Tyr Ser Phe
            85              90              95
Thr Val Gly Gly Ser Glu Ile Leu Phe Glu Val Pro Glu Val Thr Val
            100             105             110
Ala Pro Val His Ile Cys Thr Ser Trp Glu Ser Ala Ser Gly Ile Val
        115             120             125
Glu Phe Trp Val Asp Gly Lys Pro Arg Val Arg Lys Ser Leu Lys Lys
    130             135             140
Gly Tyr Thr Val Gly Ala Glu Ala Ser Ile Ile Leu Gly Gln Glu Gln
145             150             155             160
Asp Ser Phe Gly Gly Asn Phe Glu Gly Ser Gln Ser Leu Val Gly Asp
            165             170             175
Ile Gly Asn Val Asn Met Trp Asp Phe Val Leu Ser Pro Asp Glu Ile
            180             185             190
Asn Thr Ile Tyr Leu Gly Gly Pro Phe Ser Pro Asn Val Leu Asn Trp
            195             200             205
Arg Ala Leu Lys Tyr Glu Val Gln Gly Glu Val Phe Thr Lys Pro Gln
    210             215             220
Leu Trp Pro
225
```

```
<210>  4
<211>  227
<212>  PRT
<213>  Artificial Sequence
<400>  4
Met Lys Gln Ser Thr Ile Ala Leu Ala Leu Leu Pro Leu Leu Phe Thr
1               5               10              15
Pro Val Thr Lys Ala Gln Thr Asp Met Ser Arg Lys Ala Phe Val Phe
```

```
                20                      25                      30
      Pro Lys Glu Ser Asp Thr Ser Tyr Val Ser Leu Lys Ala Pro Leu Thr
              35                      40                      45
      Lys Pro Leu Lys Ala Phe Thr Val Cys Leu His Phe Tyr Thr Glu Leu
          50                      55                      60
      Ser Ser Thr Arg Gly Tyr Ser Ile Phe Ser Tyr Ala Thr Lys Arg Gln
      65                      70                      75                      80
      Asp Asn Glu Ile Leu Ile Phe Trp Ser Lys Asp Ile Gly Tyr Ser Phe
                  85                      90                      95
      Thr Val Gly Gly Ser Glu Ile Leu Phe Glu Val Pro Glu Val Thr Val
              100                     105                     110
      Ala Pro Val His Ile Cys Thr Ser Trp Glu Ser Ala Ser Gly Ile Val
          115                     120                     125
      Glu Phe Trp Val Asp Gly Lys Pro Arg Val Arg Lys Ser Leu Lys Lys
          130                     135                     140
      Gly Tyr Thr Val Gly Ala Glu Ala Ser Ile Ile Leu Gly Gln Glu Gln
      145                     150                     155                     160
      Asp Ser Phe Gly Gly Asn Phe Glu Gly Ser Gln Ser Leu Val Gly Asp
                  165                     170                     175
      Ile Gly Asn Val Asn Met Trp Asp Phe Val Leu Ser Pro Asp Glu Ile
                  180                     185                     190
      Asn Thr Ile Tyr Leu Gly Gly Pro Phe Ser Pro Asn Val Leu Asn Trp
                  195                     200                     205
      Arg Ala Leu Lys Tyr Glu Val Gln Gly Glu Val Phe Thr Lys Pro Gln
          210                     215                     220
      Leu Trp Pro
      225


      <210>   5
      <211>   621
      <212>   DNA
      <213>   Homo sapiens
      <400>   5
      cagacagaca tgtcgaggaa ggcttttgtg tttcccaaag agtcggatac ttcctatgta      60
      tccctcaaag caccgttaac gaagcctctc aaagccttca ctgtgtgcct ccacttctac     120
      acggaactgt cctcgacccg gggtacagta ttttctcgta tgccaccaag agacaagaca     180
      atgagattct tcatattttg gtctaaggat ataggataca gttttacagt gggtgggtct     240
      gaaatattat tcgaggttcc tgaagtcaca gtagctccag tacacatttg tacaagctgg     300
      gagtccgcct cagggatcgt ggagttctgg gtagatggga agcccagggt gaggaagagt     360
      ctgaagaagg gatacactgt gggggcagaa gcaagcatca tcttggggca ggagcaggat     420
      tccttcggtg ggaactttga aggaagccag tccctggtgg gagacattgg aaatgtgaac     480
      atgtgggact ttgtgctgtc accagatgag attaacacca tctatcttgg cgggcccttc     540
      agtcctaatg tcctgaactg gcgggcactg aagtatgaag tgcaaggcga agtgttcacc     600
      aaaccccagc tgtggccctg a                                               621


      <210>   6
      <211>   621
      <212>   DNA
      <213>   Homo sapiens
      <400>   6
      cagacagaca tgtcgaggaa ggcttttgtg tttcccaaag agtcggatac ttcctatgta      60
      tccctcaaag caccgttaac gaagcctctc aaagccttca ctgtgtgcct ccacttctac     120
      acggaactgt cctcgacccg tgggtacagt attttctcgt atgccaccaa gagacaagac     180
      aatgagattc tcatattttg gtctaaggat ataggataca gttttacagt gggtgggtct     240
      gaaatattat tcgaggttcc tgaagtcaca gtagctccag tacacatttg tacaagctgg     300
      gagtccgcct cagggatcgt ggagttctgg gtagatggga agcccagggt gaggaagagt     360
      ctgaagaagg gatacactgt gggggcagaa gcaagcatca tcttggggca ggagcaggat     420
      tccttcggtg ggaactttga aggaagccag tccctggtgg gagacattgg aaatgtgaac     480
      atgtgggact ttgtgctgtc accagatgag attaacacca tctatcttgg cgggcccttc     540
      agtcctaatg tcctgaactg gcgggcactg aagtatgaag tgcaaggcga agtgttcacc     600
      aaaccccagc tgtggccctg a                                               621
```

```
<210>   7
<211>   684
<212>   DNA
<213>   Artificial Sequence
<400>   7
atgaaacaaa gcactattgc actggcactc ttaccgttac tgtttacccc tgtgacaaaa      60
gcccagacag acatgtcgag gaaggctttt gtgtttccca aagagtcgga tacttcctat     120
gtatccctca aagcaccgtt aacgaagcct ctcaaagcct tcactgtgtg cctccacttc     180
tacacggaac tgtcctcgac ccggggtaca gtattttctc gtatgccacc aagagacaag     240
acaatgagat tcttcatatt ttggtctaag gatataggat acagttttac agtgggtggg     300
tctgaaatat tattcgaggt tcctgaagtc acagtagctc cagtacacat ttgtacaagc     360
tgggagtccg cctcagggat cgtggagttc tgggtagatg ggaagcccag ggtgaggaag     420
agtctgaaga agggatacac tgtgggggca gaagcaagca tcatcttggg gcaggagcag     480
gattccttcg gtgggaactt tgaaggaagc cagtccctgg tgggagacat tggaaatgtg     540
aacatgtggg actttgtgct gtcaccagat gagattaaca ccatctatct tggcgggccc     600
ttcagtccta atgtcctgaa ctggcgggca ctgaagtatg aagtgcaagg cgaagtgttc     660
accaaacccc agctgtggcc ctga                                            684
```

```
<210>   8
<211>   684
<212>   DNA
<213>   Artificial Sequence
<400>   8
atgaaacaaa gcactattgc actggcactc ttaccgttac tgtttacccc tgtgacaaaa      60
gcccagacag acatgtcgag gaaggctttt gtgtttccca aagagtcgga tacttcctat     120
gtatccctca aagcaccgtt aacgaagcct ctcaaagcct tcactgtgtg cctccacttc     180
tacacggaac tgtcctcgac ccgtgggtac agtattttct cgtatgccac caagagacaa     240
gacaatgaga ttctcatatt ttggtctaag gatataggat acagttttac agtgggtggg     300
tctgaaatat tattcgaggt tcctgaagtc acagtagctc cagtacacat ttgtacaagc     360
tgggagtccg cctcagggat cgtggagttc tgggtagatg ggaagcccag ggtgaggaag     420
agtctgaaga agggatacac tgtgggggca gaagcaagca tcatcttggg gcaggagcag     480
gattccttcg gtgggaactt tgaaggaagc cagtccctgg tgggagacat tggaaatgtg     540
aacatgtggg actttgtgct gtcaccagat gagattaaca ccatctatct tggcgggccc     600
ttcagtccta atgtcctgaa ctggcgggca ctgaagtatg aagtgcaagg cgaagtgttc     660
accaaacccc agctgtggcc ctga                                            684
```

```
<210>   9
<211>   45
<212>   DNA
<213>   Artificial Sequence
<220>
<223>   Forward Primer for CRP Gene in Example 1
<400>   9
cacacaggaa acacatatga tgaaacaaag cactattgca ctggc                      45
```

```
<210>   10
<211>   34
<212>   DNA
<213>   Artificial Sequence
<220>
<223>   Reverse Primer for CRP Gene in Example 1
<400>   10
tctagaacta gtggatcctc agggccacag ctgg                                  34
```

**Description**

Technical Field

The present invention relates to C-reactive proteins (hereinafter also referred to as "CRPs") produced by a genetic recombination technique, and use of the same. More specifically, the present invention relates to recombinant CRPs obtained by transforming the N-terminal structure of CRPs, a calibrator using the recombinant CRPs, control serum using the recombinant CRPs, and a method for quantifying CRPs based on an antibody-antigen reaction.

Background Art

CRP is a protein that exhibits a precipitation reaction with the C-polysaccharide in the pneumococcal capsule, and is also known as a typical inflammatory marker because it is a type of acute phase protein. The blood concentration of CRP increases remarkably in infectious diseases and inflammatory diseases, and decreases sharply with the recovery of symptoms. Accordingly, the quantification of CRP is used as an index to determine the severity of various diseases and to observe the course of treatment. The CRP concentration in the blood of healthy people is generally 0.3 mg/dL or less, whereas in patients with inflammation or inflammatory diseases, the CRP concentration rapidly increases hundreds to thousands of times in a short period of time. Therefore, in the measurement of CRP in a sample, it is required to accurately measure a wide range of CRP concentration from low concentration to high concentration.

As a method for quantifying blood CRP concentrations in the clinical laboratory field, there is a measurement method using an antigen-antibody reaction, and known methods are enzyme immunoassay, luminescent immunoassay, latex turbidimetric immunoassay, immunochromatography, and the like. In particular, among these measurement methods, latex turbidimetric immunoassay is widely used for daily inspections because it is easy to operate and the measurement can be automated by an analyzer. In latex turbidimetric immunoassay, the blood CRP concentration is quantified from a calibration curve using a calibrator with a known concentration. The accuracy of the calibration curve is ensured by measuring control serum.

Although natural CRP purified from human body fluids such as ascites fluid is used in the above-mentioned calibrator and control serum, there is a risk that serum components other than CRP may contaminate them and affect the measurement of blood CRP concentration. In addition, there is a risk of secondary pathogenic infection due to the handling of human body fluids as biological raw materials in the isolation and purification stages of CRP. There are also safety issues in production. Furthermore, the CRP content in human body fluids is variable, which causes a problem in terms of stable supply. On the other hand, recombinant CRP using microorganisms or the like does not use human body fluids as raw materials, and thus does not carry the risk of contamination with human-derived serum components or secondary infections. Accordingly, if a stable expression system for recombinant protein can be constructed using gene engineering technology, the desired protein can be stably supplied.

Regarding the expression of recombinant CRP by microorganisms, successful examples in *Escherichia coli,* yeast, etc., have already been reported (PTL 1 and NPL 1). However, the measurement of the recombinant CRP concentration by latex turbidimetric immunoassay had a problem that the measured values were lower than the actual CRP concentration in a high CRP concentration range. Therefore, in order to use recombinant CRP as a diagnostic raw material for a calibrator, control serum, etc., it is necessary to improve the accuracy of measurements in a high CRP concentration range.

Many proteins undergo chemical characterization or structural transformation by post-translational modifications. One of the post-translational modifications is pyroglutamylation in which the carboxyl group and amino group of glutamine or glutamine acid are converted into pyroglutamic acid by an intramolecular condensation reaction. Plants and animals have many pyroglutamyl peptides modified by pyroglutamylation, and there are proteins such as β-amyloid, collagen, and $IgG_2$. CRP is also a pyroglutamyl peptide. However, it has been reported that recombinant CRP includes those whose N-terminal is still glutamine, and those whose N-terminal is converted into pyroglutamic acid (NPL 2). There has been no report on the relationship between the N-terminal structure of CRP and the antibody-antigen reaction in latex turbidimetric immunoassay.

Citation List

Patent Literature

PTL 1: JP2000-14388A

Non-patent Literature

NPL 1: Toshio Tanaka et al., Biochem. Biophys. Res. Commun., 295 (2002), pp. 163-166
NPL 2: Journal of Clinical Laboratory Medicine, Vol. 46, No. 9, pp. 973-981 (September 2002)

Summary of Invention

Technical Problem

An object of the present invention is to improve the accuracy of measurements in a high CRP concentration range particularly when using latex turbidimetric immunoassay.

Solution to Problem

As a result of extensive research in view of the above circumstances, the present inventors found a way to solve the above problems by transforming the N-terminal structure of recombinant CRPs. Specifically, the present inventors found that it is possible to accurately measure the CRP concentration in a high CRP concentration range by using recombinant CRPs, 55% or more of which have a pyroglutamylated N-terminal, as measured by intact MS. Thus, the present invention has been completed.

Specific aspects of the present invention are as shown below.

Item 1. Recombinant C-reactive proteins produced by genetic recombination, 55% or more of the C-reactive proteins having a pyroglutamylated N-terminal.

Item 2. The recombinant C-reactive proteins according to Item 1, wherein 65% or more of the C-reactive proteins have a pyroglutamylated N-terminal.

Item 3. The recombinant C-reactive proteins according to Item 1, wherein 75% or more of the C-reactive proteins have a pyroglutamylated N-terminal.

Item 4. The recombinant C-reactive proteins according to Item 1, wherein 85% or more of the C-reactive proteins have a pyroglutamylated N-terminal.

Item 5. The recombinant C-reactive proteins according to any one of Items 1 to 4, wherein the recombinant C-reactive proteins are bacterial recombinant proteins.

Item 6. The recombinant C-reactive proteins according to Item 5, wherein the bacterium is *Escherichia coli.*

Item 7. The recombinant C-reactive proteins according to any one of Items 1 to 6, wherein the C-reactive proteins are derived from a human.

Item 8. The recombinant C-reactive proteins according to any one of Items 1 to 7, wherein the C-reactive proteins comprise any of the following polypeptides (a) to (c):

(a) a polypeptide represented by SEQ ID No: 1 or SEQ ID No: 2;
(b) a polypeptide comprising an amino acid sequence including substitution, deletion, insertion, and/or addition of one or more amino acid residues in the amino acid sequence represented by SEQ ID No: 1 or SEQ ID No: 2, and having antigenicity against anti-C-reactive protein antibody; and
(c) a polypeptide comprising an amino acid sequence having 90% or more identity to the amino acid sequence represented by SEQ ID No: 1 or SEQ ID No: 2, and having antigenicity against anti-C-reactive protein antibody.

Item 9. A calibrator comprising the recombinant C-reactive proteins according to any one of Items 1 to 8.

Item 10. Control serum comprising the recombinant C-reactive proteins according to any one of Items 1 to 8.

Item 11. A method for quantifying C-reactive proteins in a sample using the calibrator comprising the recombinant C-reactive proteins according to Item 9.

Item 12. A method for quantifying C-reactive proteins in a sample using the control serum comprising the recombinant C-reactive proteins according to Item 10.

Item 13. The method for quantifying C-reactive proteins in a sample according to Item 11 or 12, by latex turbidimetric immunoassay using latex particles on which anti-C-reactive protein antibody is immobilized.

Advantageous Effects of Invention

The recombinant CRPs of the present invention can improve the accuracy of measurements by latex turbidimetric immunoassay in a high CRP concentration range, and is useful as diagnostic raw materials for use in control serum or calibrators.

Brief Description of Drawings

Fig. 1 shows the results of performing SDS-PAGE of recombinant CRP1 in Example 3.
Fig. 2 shows the results of performing SDS-PAGE of recombinant CRP2 in Example 3.
Fig. 3 shows the results of performing comparative study of expanded spectra of average 10-valent ions with an elution time of 1.7 to 2.0 minutes in LC/MS of various types of CRP1 in Example 5.
Fig. 4 shows the results of performing comparative study of expanded spectra of average 10-valent ions with an elution time of 1.7 to 2.0 minutes in LC/MS of various types of CRP2 in Example 5.

Description of Embodiments

Polypeptide of C-Reactive Proteins

Examples of the recombinant CRPs of the present invention include CRPs derived from mammals, such as humans, dogs, cats, mice, rats, rabbits, or goats. Preferred among these are CRPs derived from humans, dogs, or cats; and particularly preferred are CRPs derived from humans.

An embodiment of the present invention is CRPs comprising any of the following polypeptides (a) to (c):

(a) a polypeptide represented by SEQ ID No: 1 or SEQ ID No: 2;
(b) a polypeptide comprising an amino acid sequence including substitution, deletion, insertion, and/or addition of one or more amino acid residues in the amino acid sequence represented by SEQ ID No: 1 or SEQ ID No: 2, and having antigenicity against anti-C-reactive protein antibody; and
(c) a polypeptide comprising an amino acid sequence having 90% or more identity to the amino acid sequence represented by SEQ ID No: 1 or SEQ ID No: 2, and having antigenicity against anti-C-reactive protein antibody.

In the polypeptide (a), SEQ ID No: 1 or SEQ ID No: 2 is an amino acid sequence of mature CRP consisting of 206 amino acids. When the recombinant CRPs of the present invention are expressed outside bacteria, such as Gram-negative bacteria, an appropriate secretion signal suitable for the host may be added. The amino acid sequence in that case has a total length of 227 amino acids, as shown in SEQ ID No: 3 or SEQ ID No: 4. Among these, 206 amino acids from positions 22 to 227 correspond to the mature CRP of SEQ ID No: 1 or SEQ ID No: 2, and the sequence from methionine to position 21 is the amino acid sequence of the secretion signal. When the recombinant CRPs of the present invention are expressed inside bacteria, the secretion signal may be deleted.

The recombinant CRPs of the present invention are not limited to (a) above, and may be those comprising:

(b) a polypeptide comprising an amino acid sequence including substitution, deletion, insertion, and/or addition of one or more amino acid residues in the amino acid sequence represented by SEQ ID No: 1 or SEQ ID No: 2, and having antigenicity against anti-C-reactive protein antibody; or
(c) a polypeptide comprising an amino acid sequence having 90% or more identity to the amino acid sequence represented by SEQ ID No: 1 or SEQ ID No: 2, and having antigenicity against anti-C-reactive protein antibody.

In the polypeptide (b), the lower limit of the number of "more amino acid residues" is 2. The upper limit is not particularly limited as long as the antigenicity against anti-C-reactive protein antibody can be maintained; however, it is necessary to be within a range in which the three-dimensional structure of the protein of amino acid residues and the antigenicity against anti-C-reactive protein antibody are not significantly impaired. For example, the upper limit is a number corresponding to less than 20% of all amino acids, preferably less than 15%, more preferably less than 10%, even more preferably less than 5%, and still even more preferably less than 1%. In other words, the number of amino acid residues is, for example, 41 or less, preferably 31 or less, more preferably 21 or less, even more preferably 10 or less, still even more preferably 5 or less, still even more preferably 4 or less, and further still even more preferably 3 or less.

In the polypeptide (c), the identity to the amino acid sequence shown in (a) is preferably 80% or more. The identity is preferably 85% or more, more preferably 90% or more, even more preferably 95% or more, still even more preferably 98% or more, and further still even more preferably 99% or more.

The amino acid sequence identity can be calculated using analysis tools available commercially or via telecommunication lines (the internet). In the present invention, the identity is calculated by selecting blastp on the BLAST website, which is a homology search program published by the National Center for Biotechnology Information (NCBI), and using the default parameters.

Whether a polypeptide has antigenicity against anti-C-reactive protein antibody is determined based on whether latex particles undergo aggregation, and the CRP concentration can be measured in the "Method for Measuring Concentration of C-Reactive Proteins" described later.

The variant of the protein having antigenicity against anti-C-reactive protein antibody and its gene can be obtained, for example, by modifying the base sequence encoding the amino acid represented by SEQ ID No: 1 or SEQ ID No: 2 using a PCR method and a commercially available kit, such as Transformer Mutagenesis Kit produced by Clontech, EXOIII/Mung Bean Deletion Kit produced by Stratagene, QuickChang Site-Directed Mutagenesis Kit produced by Stratagene, or KOD-Plus-Mutagenesis Kit produced by Toyobo Co., Ltd. The antigenicity of the protein encoded by the obtained gene can be confirmed, for example, by introducing the obtained gene into *Escherichia coli* to prepare a transformant, culturing the transformant to generate protein, and measuring the transformant, a disrupted cell suspension of the transformant, or purified protein by the "Method for Measuring Concentration of C-Reactive Proteins" described later.

DNA of C-Reactive Proteins

Examples of the DNA encoding the recombinant CRPs of the present invention include DNAs encoding CRPs derived from mammals, such as humans, dogs, cats, mice, rats, rabbits, or goats. Preferred among these are DNAs encoding CRPs derived from humans, dogs, or cats; and particularly preferred are DNAs encoding CRPs derived from humans.

An embodiment of the present invention is CRPs comprising any of the following DNAs (d) to (f):

(d) DNA encoding the amino acid sequence of the CRPs of any of (a) to (c);
(e) DNA comprising a base sequence represented by SEQ ID No: 5 or SEQ ID No: 6;
(f) DNA encoding a polypeptide comprising a base sequence including substitution, deletion, insertion, and/or addition of one or more bases in the base sequence represented by SEQ ID No: 5 or SEQ ID No: 6, and having antigenicity against anti-C-reactive protein antibody; and
(g) DNA encoding a polypeptide comprising a base sequence having 80% or more identity to the base sequence represented by SEQ ID No: 5 or SEQ ID No: 6, and having antigenicity against anti-C-reactive protein antibody.

In the DNA (d), the amino acid sequence of the CRPs of the present invention is the amino acid sequence of the CRPs shown in any of (a) to (c). The options for the DNA of the present invention are not particularly limited when there are multiple codons corresponding to the amino acids in the amino acid sequence. Specific examples include (e) DNA comprising the base sequence represented by SEQ ID No: 5 or SEQ ID No: 6.

When a secretion signal sequence is added as described above, it is preferable to use DNA represented by SEQ ID No: 7 or SEQ ID No: 8. SEQ ID No: 7 or SEQ ID No: 8 encodes the mature CRP with 206 amino acids from positions 22 to 227 in the total length of CRP represented by the amino acid sequence of SEQ ID No: 3 or SEQ ID No: 4, and a part corresponding to the secretion signal from methionine to position 21.

The DNA of the present invention is not limited to those mentioned above, and may be:

(f) DNA encoding a polypeptide comprising a base sequence including substitution, deletion, insertion, and/or addition of one or more bases in the base sequence represented by SEQ ID No: 5 or SEQ ID No: 6, and having antigenicity against anti-C-reactive protein antibody; or
(g) DNA encoding a polypeptide comprising a base sequence having 80% or more identity to the base sequence represented by SEQ ID No: 5 or SEQ ID No: 6, and having antigenicity against anti-C-reactive protein antibody.

When the DNA encoding the CRPs of the present invention is incorporated into a host other than organisms derived from *Escherichia coli* or the like to express the CRPs of the present invention, the base sequence may be changed according to the codon usage of the host organism, in order to improve the expression efficiency.

In the DNA (f), the lower limit of the number of "more bases" is 2. The upper limit is not particularly as long as the antigenicity against anti-C-reactive protein antibody of the polypeptide encoded by the DNA can be maintained; however, it is necessary to be within a range in which the three-dimensional structure of the protein of amino acid residues and the antigenicity against anti-C-reactive protein antibody are not significantly impaired. For example, the upper limit is a number corresponding to less than 20% of all amino acids of the polypeptide before modification, preferably less than 15%, more preferably less than 10%, even more preferably less than 5%, and still even more preferably less than 1%. In other words, the number of bases is, for example, 124 or less (the number of bases corresponding to 20% of all amino acids), preferably 93 or less (15%), more preferably 62 or less (10%), even more preferably 31 or less (5%), still even more preferably 20 or less, still even more preferably 15 or less, still even more preferably 10 or less, still even more preferably 5 or less, still even more preferably 4 or less, and further still even more preferably 3 or less.

In the DNA (g), the identity to the base sequence represented by SEQ ID No: 5 or SEQ ID No: 6 is preferably 80% or more, more preferably 85% or more, even more preferably 90% or more, still even more preferably 95% or more, still even more preferably 98% or more, and further still even more preferably 99% or more.

The base sequence identity can be calculated using analysis tools available commercially or via telecommunication lines (the internet). In the present invention, the identity is calculated by selecting blastn on the BLAST website, which is a homology search program published by the National Center for Biotechnology Information (NCBI), and using the default parameters.

Whether a DNA encodes a polypeptide having antigenicity against anti-C-reactive protein antibody can be determined in such a manner that the DNA is incorporated into a commercial expression vector and expressed in a suitable host, and the antigenicity against anti-C-reactive protein antibody of the obtained polypeptide can be determined based on whether latex particles undergo aggregation, and the CRP concentration can be measured in the "Method for Measuring Concentration of C-Reactive Proteins" described later.

Method for Producing C-Reactive Proteins

Another embodiment of the present invention is a vector into which the above DNA is incorporated, a transformant comprising the vector, or a method for producing recombinant CRPs, comprising culturing the transformant. The recombinant CRPs of the present invention can be easily prepared by inserting the gene into a suitable vector to prepare a recombinant vector, and transforming a suitable host cell with the recombinant vector to prepare a transformant, and culturing the transformant.

The vector is not particularly limited as long as it can replicate and retain or autonomously proliferate in various host cells of prokaryotic and/or eukaryotic cells. Examples include plasmid vectors, phage vectors, viral vectors, and the like. The preparation of the recombinant vector is not particularly limited, and may be performed according to a general method. For example, the preparation can easily be performed by linking the gene of the CRPs of the present invention to such a vector using an appropriate restriction enzyme and ligase or, if necessary, further a linker or adaptor DNA. A gene fragment amplified using a DNA polymerase that adds a single base to the amplified end, such as Taq DNA polymerase, can also be connected to the vector by TA cloning.

Moreover, the host cell is not particularly limited as long as a recombinant expression system is established. Preferred examples include microorganisms, such as *Escherichia coli, Bacillus subtilis,* actinomycetes, *Aspergillus oryzae,* and yeast, as well as insect cells, animal cells, higher plants, and the like. More preferred are microorganisms, and particularly preferred is *Escherichia coli* (e.g., K12 strain and B strain). The preparation of the transformant is not particularly limited, and may be performed according to a general method. When the host is *Escherichia coli, Escherichia coli* C600, *Escherichia coli* HB101, *Escherichia coli* DH5α, *Escherichia coli* JM109, *Escherichia coli* BL21, or the like is used. Examples of vectors include pBR322, pUC19, pBluescript, pQE, pET, and the like. When the host is yeast, preferred examples include *Saccharomyces cerevisiae, Schizosaccharomyces pombe, Candida utilis, Pichia pastoris,* and the like. Examples of vectors include pAUR101, pAUR224, pYE32, and the like. When the host is a filamentous fungus, examples include *Aspergillus oryzae, Aspergillus niger,* and the like.

When the obtained transformant is cultured for a certain period of time under culture conditions suitable for the host cell, the recombinant CRPs of the present invention are expressed by the incorporated gene and accumulated in the transformant.

The recombinant CRPs of the present invention accumulated in the transformant can be used in an unpurified form; however, it is preferable to use a purified form. The purification method is not particularly limited, and can be performed, for example, by homogenizing the transformant after culture or a cultured product thereof in a suitable buffer, obtaining a cell extract by sonication, surfactant treatment, or the like, and then appropriately combining separation techniques commonly used for protein separation and purification. Examples of such separation techniques include, but are not limited to, methods using the difference in solubility, such as salting out and solvent precipitation; methods using the difference in molecular weight, such as dialysis, ultrafiltration, gel filtration, unmodified polyacrylamide gel electrophoresis (PAGE), and sodium dodecyl sulfate-polyacrylamide gel electrophoresis (SDS-PAGE); methods using charges, such as ion exchange chromatography and hydroxyapatite chromatography; methods using specific affinity, such as affinity chromatography using a phosphorylcholine-immobilized column; methods using the difference in hydrophobicity, such as reverse phase high-performance liquid chromatography; methods using the difference in isoelectric point, such as isoelectric focusing electrophoresis; and the like. For example, purified preparations can be obtained by separation by gel filtration using Sephadex gel (produced by GE Healthcare Bio-Sciences Corp) or the like, or column chromatography using DEAE Sepharose CL-6B (produced by GE Healthcare Bio-Sciences Corp), Octyl-Sepharose CL-6B (produced by GE Healthcare Bio-Sciences Corp), or the like, and purification.

Method for Cyclizing N-Terminal of C-Reactive Proteins

55% or more of all of the recombinant CRPs of the present invention are recombinant CRPs with a pyroglutamylated N-terminal, and specifically recombinant CRPs with an N-terminal cyclization rate calculated as 55% or more.

In the present invention, the "N-terminal cyclization rate" is an indicator showing the ratio of recombinant CRPs with a pyroglutamylated N-terminal among recombinant CRPs, and is calculated according to the "Method for Measuring N-Terminal Cyclization Rate of C-Reactive Proteins" described later. When the N-terminal cyclization rate is calculated by the "Method for Measuring N-Terminal Cyclization Rate of C-Reactive Proteins" described later, it is preferable to hold 55% or more of recombinant CRPs, preferably 60% or more, more preferably 65% or more, even more preferably 70% or more, still even more preferably 75% or more, still even more preferably 80% or more, still even more preferably 85% or more, still even more preferably 90% or more, still even more preferably 95% or more, and further still even more preferably 98% or more.

In the present invention, the "N-terminal" refers to the N-terminal of mature CRP from which a secretion signal sequence is removed, and specifically refers to glutamine at position 1 of the amino acid sequence represented by SEQ ID No: 1 or SEQ ID No: 2 (or at position 22 of SEQ ID No: 3 or SEQ ID No: 4), or pyroglutamyl acid after the glutamine at position 1 undergoes an intramolecular condensation reaction. The glutamine at position 1 is converted into pyroglutamic acid by cyclization treatment.

In the present invention, the "cyclization treatment" refers to a treatment that promotes the conversion of CRP from a state where the above N-terminal is glutamine (hereinafter referred to as "uncyclized") to a state where the above N-terminal is pyroglutamyl acid (hereinafter referred to as "cyclized"). An enzymatic or non-enzymatic method may be selected. The enzymatic method can be performed, for example, by reacting enzyme, such as glutaminyl cyclase, at an appropriate temperature; however, the enzyme and temperature condition are not limited thereto. For the non-enzymatic method, it is necessary to set the type of buffer, pH, treatment temperature, treatment time, CRP concentration, etc.; however, any conditions that do not adversely affect CRP, uncyclized CRP, and cyclized CRP may be used, and these are not particularly limited. An example is shown below.

Examples of the buffer used in the cyclization treatment include Good's buffers, such as acetic acid buffer, MES buffer, and PIPES buffer; phosphoric acid buffer, Tris-HCl buffer, boric acid buffer, glycine buffer, and the like. The pH condition is preferably in the range of pH 7 to pH 12, and more preferably pH 7 to pH 10. The temperature condition is preferably 4°C to 55°C, and more preferably 37°C to 50°C. The CRP concentration is preferably 0.1 mg/dL to 500 mg/dL, and more preferably 10 mg/dL to 300 mg/dL. The reaction time is preferably 30 minutes to 4 weeks, and more preferably 16 hours to 3 weeks.

Method for Measuring Concentration of C-Reactive Proteins

In the present invention, the CRP concentration is measured under the following conditions. In the CRP concentration measurement method, latex particles with immobilized anti-C-reactive protein antibody and CRP (test substance) in a test sample cause an antigen-antibody reaction, and the CRP concentration is measured from the degree of aggregation of the latex particles. In the present invention, the "CRP concentration" refers to a value measured by the following method, unless otherwise specified.

Reagents

- CRP latex X2 "Seiken" R1 reagent (buffer), produced by Denka Seiken Co., Ltd.
- CRP latex X2 "Seiken" R2 reagent (latex (anti-human CRP polyclonal antibody (rabbit)-bound latex) suspension), produced by Denka Seiken Co., Ltd.
- CRPX2 Standard Liquid H, produced by Denka Seiken Co., Ltd.

Measurement sample

The measurement sample is a CRP solution, and is diluted with 20 mM Tris-HCl buffer (0.14 M sodium chloride, 2 mM calcium chloride; pH 7.5), as needed, before use. When a sample with a specific CRP concentration is prepared, the dilution factor is determined based on the protein concentration value-assigned in the Bradford protein assay.

Measurement method

Using the above measurement sample, and the above R1 reagent and R2 reagent, the CRP concentration (mg/dL) of the sample is measured under the following conditions using Hitachi 7180 Fully Automatic Biochemistry Analyzer. The CRP concentration is calculated by formula (I).

Sample: 2.2 μL
R1 reagent: 120 μL
R2 reagent: 120 μL
Measurement method: two-point end method (18-34)
Dominant wavelength: 546 nm
Complementary wavelength: 800 nm

```
CRP concentration (mg/dL) = {measured value (TEST) - measured

value (BLANK)} x dilution factor of CRP solution . . . (I)
```

In the present invention, as the method for determining whether the reactivity of the recombinant CRPs to the latex reagents in a high CRP concentration range is improved, when the deviation between the CRP concentration value of natural human CRP in the high CRP concentration range measured by the above method, and the CRP concentration value of each recombinant CRP in the high CRP concentration range is 5% or less, it is determined that the reactivity is improved. The high CRP concentration range in the present invention refers to the CRP concentration range of 10 to 30 mg/dL, but is not particularly limited thereto.

Method for Measuring N-Terminal Cyclization Rate of C-Reactive Proteins

In the present invention, the N-terminal cyclization rate of the recombinant CRPs is measured under the following conditions. In this measurement method, the 10-valent ion intensities of the spectra of uncyclized CRP and cyclized CRP are measured by mass spectrometry, and the ratio of uncyclized CRP and cyclized CRP is calculated from each ion intensity. In the present invention, the "spectrum of uncyclized CRP" refers to a spectrum corresponding to a molecular weight of 23045, and the "spectrum of cyclized CRP" refers to a spectrum corresponding to a molecular weight of 23027. In the present invention, the "N-terminal cyclization rate of CRP" specifically refers to a value measured by the following method, unless otherwise specified.

Measurement sample

The measurement sample is a CRP solution, and is diluted with ultrapure water, as needed, before use.

Measurement method

The spectra of uncyclized CRP and cyclized CRP in the above measurement sample are measured under the following conditions using LC/MS devices.

LC conditions

Device: ACQUITY UPLC, produced by Waters
Column: MassPREP Micro Desalting Column (20 μm, 2.1 x 5 mm), produced by Waters
Mobile phase:

A: water/formic acid mixture (1000:1),
B: IPA/ACN/methanol/formic acid mixture (500:300:200:1)

Column temperature: 50°C
Injection amount: 5 μL
MS conditions
Device: micrOTOF, produced by Bruker Daltonics

Ionization method: ESI Positive

Method for calculating N-terminal cyclization rate

The 10-valent ion intensity of the average spectrum with an elution time of 1.7 to 2.0 minutes for each CRP spectrum obtained by the above measurement method is used to calculate the ratio of uncyclized CRP and cyclized CRP. The "ratio" in the present invention refers to the ratio of the ion intensity of cyclized CRP when the sum of the two ion intensities of uncyclized CRP and cyclized CRP is 100, and is calculated by the following formula (II).

```
N-terminal cyclization rate (%) = ion intensity of cyclized
CRP/(ion intensity of uncyclized CRP + ion intensity of cyclized
CRP) x 100 . . . (II)
```

The present invention is described in more detail below with reference to Examples. The present invention is not particularly limited by the Examples.

Example 1: Introduction of Variation and Acquisition of Transformant

(1) Introduction of Variation

An artificial synthetic gene of SEQ ID No: 7 or SEQ ID No: 8 obtained by linking *Escherichia* coli-derived alkaline phosphatase secretion signal sequence (ATGAAACAAAGCACTATTGCACTGGCACTCTTACCGTTACTGTTTACCCCTGTGACAAAAGCC) and human-derived mature CRP sequence of SEQ ID No: 5 or SEQ ID No: 6 was used as a template, and primers of SEQ ID Nos: 9 and 10 were used to amplify a CRP gene. SEQ ID No: 9 is a forward primer, and SEQ ID No: 10 is a reverse primer. Restriction enzyme site NdeI or restriction enzyme site BamHI is added to the primers. The amplified gene fragment, vector plasmid pBluescript KSN(+) cleaved with the restriction enzymes NdeI and BamHI, and In-Fusion Reaction Mix (produced by Takara Bio Inc.) were added, followed by incubation, thereby constructing a plasmid. In this manner, recombinant plasmid pBKSN_CRP1 including SEQ ID No: 7 and recombinant plasmid pBKSN_CRP2 including SEQ ID No: 8, which were designed to express a large amount of CRP gene, were obtained.

(2) Acquisition of Transformant

Using the plasmid constructed in (1), *Escherichia coli* JM109 strain competent cells (produced by Toyobo Co., Ltd.) were transformed, cultured in SOC medium for 1 hour at 37°C, and then developed on LB agar medium (containing 1.0% glucose and 50 μg/mL ampicillin) to obtain a colony as a transformant. The transformant obtained by introducing pBKSN_CRP1 was named *Escherichia coli* JM109 (pBKSN_CRP1). Further, a transformant obtained by introducing pBKSN_CRP2 in the same manner as above was named *Escherichia coli* JM109 (pBKSN_CRP2).

Example 2: Expression of CRP Gene in *Escherichia coli*

The colony of the transformant *Escherichia coli* JM109 (pBKSN_CRP1) obtained in Example 1 was inoculated in 5 mL of LB liquid medium (containing 1.0% glucose and 100 μg/mL ampicillin) sterilized *in vitro,* and cultured at 37°C for 16 hours. The obtained culture broth was used as a seed culture broth and inoculated in 500 mL of LB liquid medium (containing 0.5% glycerol, 0.05% calcium chloride, 1 mM IPTG, and 50 μg/mL ampicillin) in 10 2-L Sakaguchi flasks. Then, the cells were cultured at a shaking speed of 180 rpm at 30°C for 24 hours. At the end of the culture, the bacterial bodies were collected by centrifugation, suspended in 20 mM Tris-HCl buffer (0.14 M sodium chloride, 2 mM calcium chloride, pH 7.5), then crushed in a French press (produced by Niro Soavi), and further centrifuged to thereby obtain the supernatant liquid as a crude purified liquid 1. In addition, a crude purified liquid 2 was obtained from *Escherichia coli* JM109 (pBKSN_CRP2) in the same manner as above.

Example 3: Purification of Recombinant CRPs

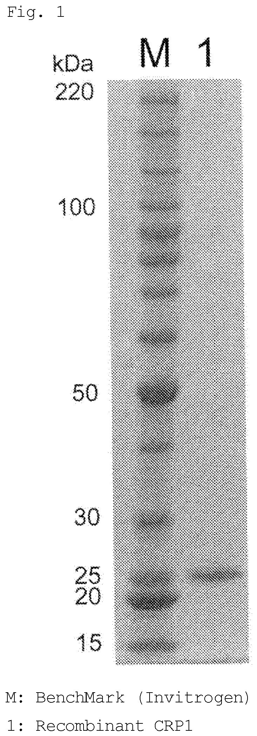

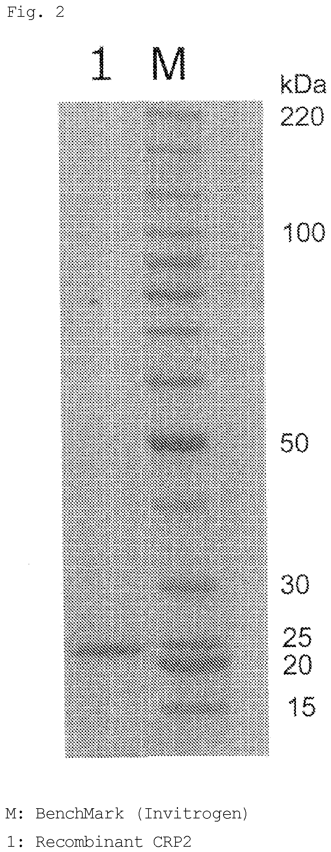

The crude purified liquid 1 obtained in Example 2 was subjected to affinity purification using Pierce™ p-Aminophenyl Phosphoryl Choline Agarose (produced by Thermo Scientific). The resin equilibrated with the buffer used in Example 2, i.e., 20 mM Tris-HCl buffer (0.14 M sodium chloride, 2 mM calcium chloride, pH 7.5), was mixed and absorbed with the crude purified liquid. The resulting resin was washed with the above buffer, and eluted in 20 mM Tris-HCl buffer (0.14 M sodium chloride, 2 mM EDTA, pH 7.5) to obtain a recombinant CRP solution 1. The solution was further replaced with the buffer used in Example 2, i.e., 20 mM Tris-HCl buffer (0.14 M sodium chloride, 2 mM calcium chloride, pH 7.5), while removing EDTA by water concentration using a hollow fiber membrane, and further concentrated to a suitable concentration with a centrifugal ultrafiltration filter (produced by Merck), thereby obtaining high-purity recombinant CRP1. Fig. 1 shows the results of performing SDS-PAGE using the obtained recombinant CRP1. As a result, improving the purity to a level at which impure protein could not be detected by SDS-PAGE succeeded. In addition, high-purity recombinant CRP2 was also obtained from the crude purified liquid 2 in the same manner as above. Fig. 2 shows the results of performing SDS-PAGE on the obtained CRP2. As in the case of CRP1, improving the purity to a level at which impure protein could not be detected by SDS-PAGE succeeded.

Example 4: N-Terminal Cyclization Treatment of Recombinant CRPs

The recombinant CRP1 obtained in Example 3 was heated at 37°C for 8 days to obtain cyclized recombinant CRP1 in which the N-terminal of recombinant CRP was cyclized by pyroglutamylation. In the cyclization treatment, the buffer used in Example 2, i.e., 20 mM Tris-HCl buffer (0.14 M sodium chloride, 2 mM calcium chloride, pH 7.5), was used, and the CRP concentration was 300 mg/dL. Further, cyclized recombinant CRP2 was obtained from the recombinant CRP2 in the same manner as above.

Example 5: Measurement of N-Terminal Cyclization Rate

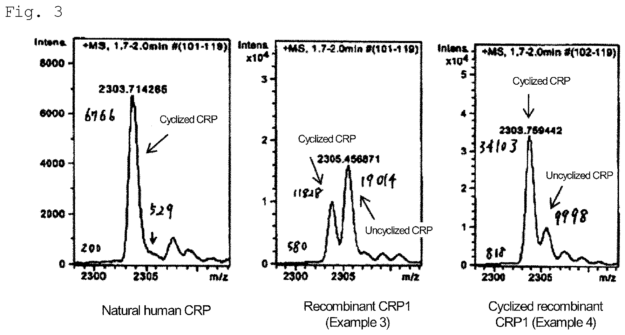

Using the recombinant CRP1 and recombinant CRP2 obtained in Example 3, the cyclized recombinant CRP1 and cyclized recombinant CRP2 obtained in Example 4, and natural human CRP (produced by Yashraj) as a positive control, CRP solutions were prepared using ultrapure water to a CRP concentration of 150 mg/dL, followed by measurement under the LC/MS conditions as described above (see Table 1). Fig. 3 shows an enlargement of the average 10-valent ion with an elution time of 1.7 to 2.0 minutes in the mass spectrum of each sample of recombinant CRP1.

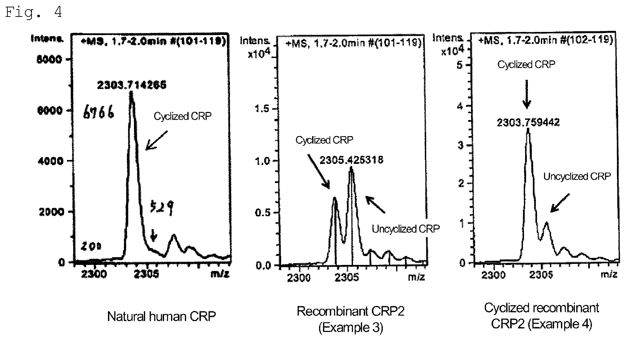

m/z 2305.45 represents the spectrum of uncyclized CRP, and m/z 2303.74 represents the spectrum of cyclized CRP. In the natural human CRP, the spectrum of cyclized CRP was shown prominently. On the other hand, in the recombinant CRP1 obtained in Example 3, it was shown that the spectrum of uncyclized CRP was higher than the spectrum of cyclized CRP. In contrast, in the cyclized recombinant CRP1 obtained in Example 4, it was shown that the spectrum of cyclized CRP was clearly higher than the spectrum of uncyclized CRP. It was shown that the N-terminal cyclization treatment of Example 4 promoted the pyroglutamylation of CRP. Similar results were obtained for the recombinant CRP2 and the cyclized recombinant CRP2, as shown in Fig. 4.

Table 1

| Time (min) | Flow rate (mL/min) | Mobile phase B (%) |
|---|---|---|
| 0.01 | 0.40 | 5 |
| 0.50 | 0.40 | 5 |
| 0.51 | 0.16 | 5 |
| 2.00 | 0.16 | 95 |
| 2.10 | 0.40 | 5 |
| 2.70 | 0.40 | 95 |
| 2.80 | 0.40 | 5 |
| 3.40 | 0.40 | 95 |
| 3.50 | 0.40 | 5 |
| 4.00 | 0.40 | 5 |

In the recombinant CRP1 and recombinant CRP2 obtained in Example 3, the cyclized recombinant CRP1 and cyclized recombinant CRP2 obtained in Example 4, and natural human CRP, the N-terminal cyclization rate of each CRP was calculated by the above formula (II) from the ratio of uncyclized CRP and cyclized CRP from each ion intensity based on the 10-valent ion intensities of the spectra of uncyclized CRP and cyclized CRP measured under the above LC/MS conditions. Table 2 shows the N-terminal cyclization rates of the recombinant CRP1, cyclized recombinant CRP1, and natural human CRP. For the cyclized recombinant CRP1 obtained in Example 4, the N-terminal cyclization rate of the cyclized recombinant CRP1 obtained on days 1, 3, 6, and 8 of heating was calculated by formula (II). Further, Table 3 shows the N-terminal cyclization rates of the recombinant CRP2, cyclized recombinant CRP2, and natural human CRP. For the cyclized recombinant CRP2 obtained in Example 4, the N-terminal cyclization rate of the cyclized recombinant CRP2 obtained on days 1, 3, 6, and 8 of heating was calculated by formula (II).

The N-terminal cyclization rate of the natural human CRP is 95%, indicating that 95% of the total is cyclized CRP. On the other hand, the N-terminal cyclization rates of the recombinant CRP1 and recombinant CRP2 obtained in Example 3 are 40%, indicating that cyclized CRP is present in less than half of the total. In contrast, the cyclized recombinant CRP1 and cyclized recombinant CRP2 obtained in Example 4 showed an N-terminal cyclization rate of 42% on day 1 of heating, an N-terminal cyclization rate 54% on day 3 of heating, an N-terminal cyclization rate of 67% on day 6 of heating, and an N-terminal cyclization rate of 78% on day 8 of heating, indicating that the proportion of cyclized recombinant CRP tended to increase as the heating time increased.

Table 2

| Sample | N-terminal cyclization rate (%) |
|---|---|
| Example 3: recombinant CRP1 | 40 |
| Example 4: cyclized recombinant CRP1, day 1 of heating | 42 |
| Example 4: cyclized recombinant CRP1, day 3 of heating | 54 |
| Example 4: cyclized recombinant CRP1, day 6 of heating | 67 |
| Example 4: cyclized recombinant CRP1, day 8 of heating | 78 |
| Natural human CRP | 95 |

Table 3

| Sample | N-terminal cyclization rate (%) |
|---|---|
| Example 3: recombinant CRP2 | 40 |
| Example 4: cyclized recombinant CRP2, day 1 of heating | 42 |
| Example 4: cyclized recombinant CRP2, day 3 of heating | 54 |
| Example 4: cyclized recombinant CRP2, day 6 of heating | 67 |
| Example 4: cyclized recombinant CRP2, day 8 of heating | 78 |
| Natural human CRP | 95 |

Example 6: Measurement of CRP Concentration by Latex Reagent

Bovine serum albumin (produced by Sigma) was diluted with ultrapure water to 0.1, 0.2, 0.4, and 0.75 mg/mL to prepare standard solutions. 600 $\mu$L of protein assay concentrated dye reagent (produced by Bio-Rad) and 2.4 mL of ultrapure water were added to 60 $\mu$L of each standard solution, and the mixture was allowed to stand at room temperature for 5 minutes. Then, the absorbance at 595 nm was measured. A calibration curve was prepared from the measured absorbance at 595 nm and the bovine serum albumin concentration. The recombinant CRP1 and recombinant CRP2 obtained in Example 3, the cyclized recombinant CRP1 and cyclized recombinant CRP2 obtained in Example 4, and natural human CRP were diluted with ultrapure water to a suitable concentration using the same reagent under the same conditions as those for the above standard solutions, and the absorbance at 595 nm was measured. From the measured values of these CRPs, the protein concentration was calculated from the above calibration curve. Based on the above protein concentration, CRP solutions were prepared by dilution with 20 mM Tris-HCl buffer (0.14 M sodium chloride, 2 mM calcium chloride, pH 7.5) to protein concentrations of 1 mg/dL, 5 mg/dL, 10 mg/dL, and 30 mg/dL.

For the CRP concentration of each of the above CRP solutions, a first reagent (a buffer of CRP-latex X2 produced by Denka Seiken Co., Ltd.) and a second reagent (an anti-human CRP polyclonal antibody (rabbit)-bound latex suspension) were combined, and the CRP concentration-dependent formation of particle aggregates was measured using Hitachi 7180 Fully Automatic Biochemistry Analyzer. Specifically, 120 $\mu$L of the first reagent was added to 2.2 $\mu$L

of each of the CRP solutions, the mixture was heated at 37°C For 5 minutes, and then 120 μL of the second reagent was added and stirred. Thereafter, the change in absorbance (ΔmAbs) associated with the aggregate formation for 5 minutes was measured at a dominant wavelength of 546 nm and a complementary wavelength of 800 nm.

Table 4 shows the measured values of the recombinant CRP1 obtained in Example 3 and the cyclized recombinant CRP1 obtained in Example 4, and the relative values at each concentration with respect to the measured value of the natural human CRP. Table 4 also shows the N-terminal cyclization rates of the various CRPs calculated in Example 5. Furthermore, Table 5 shows the measured values of the recombinant CRP1 obtained in Example 3, the cyclized recombinant CRP1 obtained in Example 4, and the natural human CRP. As in the above case, Table 6 shows the measured values of the recombinant CRP2 obtained in Example 3 and the cyclized recombinant CRP2 obtained in Example 4, and the relative values at each concentration with respect to the measured value of the natural human CRP. Table 6 also shows the N-terminal cyclization rates of the various CRPs calculated in Example 5. Furthermore, Table 7 shows the measured values of the recombinant CRP2 obtained in Example 3, the cyclized recombinant CRP2 obtained in Example 4, and the natural human CRP.

Tables 4 and 6 confirmed that in the recombinant CRP1 and recombinant CRP2 with an N-terminal cyclization rate of 40% or 42%, the relative values with respect to the measured value of the natural human CRP at the points of 10 mg/dL and 30 mg/dL were 89 to 94%, and that there was a deviation of 6 to 11% in the measured values. On the other hand, in the cyclized recombinant CRP1 and cyclized recombinant CRP2 with an N-terminal cyclization rate of 54%, 67%, or 78%, the relative values with respect to the measured value of the natural human CRP at the points of 10 mg/dL and 30 mg/dL were 95 to 104%, and the deviation from the measured values was suppressed within 5%. These results indicated that in the cyclized recombinant CRP1 and cyclized recombinant CRP2 with an N-terminal cyclization rate of 55% or more, the deviation from the natural human CRP could be suppressed within 5% even in the high CRP concentration ranges of 10 mg/dL and 30 mg/dL.

Table 4

|  | Example 3 recombinant CRP1 | Example 4 cyclized CRP1 day 1 of heating | Example 4 cyclized CRP1 day 3 of heating | Example 4 cyclized CRP1 day 6 of heating | Example 4 cyclized CRP1 day 8 of heating |
|---|---|---|---|---|---|
| N-terminal cyclization rate | 40% | 42% | 54% | 67% | 78% |
| Theoretical value (mg/dL) | Relative value (recombinant CRP/natural human CRP) | | | | |
| 1 | 103% | 100% | 102% | 104% | 102% |
| 5 | 98% | 96% | 100% | 104% | 101% |
| 10 | 94% | 89% | 97% | 102% | 99% |
| 30 | 91% | 91% | 95% | 100% | 101% |

Table 5

|  | Example 3 recombinant CRP1 | Example 4 cyclized CRP1 day 1 of heating | Example 4 cyclized CRP1 day 3 of heating | Example 4 cyclized CRP1 day 6 of heating | Example 4 cyclized CRP1 day 8 of heating | Natural human CRP |
|---|---|---|---|---|---|---|
| N-terminal cyclization rate | 40% | 42% | 54% | 67% | 78% | 95% |
| Theoretical value (mg/dL) | Measured value (mg/dL) | | | | | |
| 1 | 0.79 | 0.76 | 0.77 | 0.79 | 0.78 | 0.76 |
| 5 | 4.80 | 4.69 | 4.88 | 5.06 | 4.92 | 4.89 |
| 10 | 9.54 | 9.01 | 9.87 | 10.38 | 10.08 | 10.14 |

(continued)

| | Example 3 recombinant CRP1 | Example 4 cyclized CRP1 day 1 of heating | Example 4 cyclized CRP1 day 3 of heating | Example 4 cyclized CRP1 day 6 of heating | Example 4 cyclized CRP1 day 8 of heating | Natural human CRP |
|---|---|---|---|---|---|---|
| 30 | 27.74 | 27.57 | 28.83 | 30.37 | 30.66 | 30.31 |

Table 6

| | Example 3 recombinant CRP2 | Example 4 cyclized CRP2 day 1 of heating | Example 4 cyclized CRP2 day 3 of heating | Example 4 cyclized CRP2 day 6 of heating | Example 4 cyclized CRP2 day 8 of heating |
|---|---|---|---|---|---|
| N-terminal cyclization rate | 40% | 42% | 54% | 67% | 78% |
| Theoretical value (mg/dL) | Relative value (recombinant CRP/natural human CRP) | | | | |
| 1 | 103% | 100% | 102% | 104% | 102% |
| 5 | 98% | 96% | 100% | 104% | 101% |
| 10 | 94% | 89% | 97% | 102% | 99% |
| 30 | 91% | 91% | 95% | 100% | 101% |

Table 7

| | Example 3 recombinant CRP2 | Example 4 cyclized CRP2 day 1 of heating | Example 4 cyclized CRP2 day 3 of heating | Example 4 cyclized CRP2 day 6 of heating | Example 4 cyclized CRP2 day 8 of heating | Natural human CRP |
|---|---|---|---|---|---|---|
| N-terminal cyclization rate | 40% | 42% | 54% | 67% | 78% | 95% |
| Theoretical value (mg/dL) | Measured value (mg/dL) | | | | | |
| 1 | 0.79 | 0.76 | 0.77 | 0.79 | 0.78 | 0.76 |
| 5 | 4.80 | 4.69 | 4.88 | 5.06 | 4.92 | 4.89 |
| 10 | 9.54 | 9.01 | 9.87 | 10.38 | 10.08 | 10.14 |
| 30 | 27.74 | 27.57 | 28.83 | 30.37 | 30.66 | 30.31 |

Industrial Applicability

The CRPs of the present invention are particularly useful in the medical and diagnostic fields as diagnostic raw materials for use in latex reagents with excellent reactivity in a high CRP concentration range.

**Claims**

1. Recombinant C-reactive proteins produced by genetic recombination, 55% or more of the C-reactive proteins having a pyroglutamylated N-terminal.

2. The recombinant C-reactive proteins according to claim 1, wherein 65% or more of the C-reactive proteins have a pyroglutamylated N-terminal.

3. The recombinant C-reactive proteins according to claim 1, wherein 75% or more of the C-reactive proteins have a pyroglutamylated N-terminal.

4. The recombinant C-reactive proteins according to claim 1, wherein 85% or more of the C-reactive proteins have a pyroglutamylated N-terminal.

5. The recombinant C-reactive proteins according to any one of claims 1 to 4, wherein the recombinant C-reactive proteins are bacterial recombinant proteins.

6. The recombinant C-reactive proteins according to claim 5, wherein the bacterium is *Escherichia coli.*

7. The recombinant C-reactive proteins according to any one of claims 1 to 6, wherein the C-reactive proteins are derived from a human.

8. The recombinant C-reactive proteins according to any one of claims 1 to 7, wherein the C-reactive proteins comprise any of the following polypeptides (a) to (c):

(a) a polypeptide represented by SEQ ID No: 1 or SEQ ID No: 2;
(b) a polypeptide comprising an amino acid sequence including substitution, deletion, insertion, and/or addition of one or more amino acid residues in the amino acid sequence represented by SEQ ID No: 1 or SEQ ID No: 2, and having antigenicity against anti-C-reactive protein antibody; and
(c) a polypeptide comprising an amino acid sequence having 90% or more identity to the amino acid sequence represented by SEQ ID No: 1 or SEQ ID No: 2, and having antigenicity against anti-C-reactive protein antibody.

9. A calibrator comprising the recombinant C-reactive proteins according to any one of claims 1 to 8.

10. Control serum comprising the recombinant C-reactive proteins according to any one of claims 1 to 8.

11. A method for quantifying C-reactive proteins in a sample using the calibrator comprising the recombinant C-reactive proteins according to claim 9.

12. A method for quantifying C-reactive proteins in a sample using the control serum comprising the recombinant C-reactive proteins according to claim 10.

13. The method for quantifying C-reactive proteins in a sample according to claim 11 or 12, by latex turbidimetric immunoassay using latex particles on which anti-C-reactive protein antibody is immobilized.

Fig. 1

M: BenchMark (Invitrogen)

1: Recombinant CRP1

Fig. 2

M: BenchMark (Invitrogen)

1: Recombinant CRP2

Fig. 3

Natural human CRP

Recombinant CRP1
(Example 3)

Cyclized recombinant
CRP1 (Example 4)

Fig. 4

| Natural human CRP | Recombinant CRP2<br>(Example 3) | Cyclized recombinant<br>CRP2 (Example 4) |

**INTERNATIONAL SEARCH REPORT**

International application No.

PCT/JP2020/039893

A. CLASSIFICATION OF SUBJECT MATTER
Int. Cl. C07K14/47(2006.01)i, C12N15/12(2006.01)i, C12N15/31(2006.01)i,
G01N33/53(2006.01)i, G01N33/543(2006.01)i, G01N33/96(2006.01)i
FI: C12N15/12, G01N33/543 581A, G01N33/96, C12N15/31, G01N33/53 X, C07K14/47 ZNA

According to International Patent Classification (IPC) or to both national classification and IPC

B. FIELDS SEARCHED

Minimum documentation searched (classification system followed by classification symbols)
Int. Cl. C07K14/47, C12N15/12, C12N15/31, G01N33/53, G01N33/543,
G01N33/96

Documentation searched other than minimum documentation to the extent that such documents are included in the fields searched
Published examined utility model applications of Japan        1922-1996
Published unexamined utility model applications of Japan      1971-2020
Registered utility model specifications of Japan             1996-2020
Published registered utility model applications of Japan     1994-2020

Electronic data base consulted during the international search (name of data base and, where practicable, search terms used)
JSTPlus/JMEDPlus/JST7580 (JDreamIII); CAplus/MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| Y | JP 6-311884 A (ORIENTAL YEAST CO., LTD.) 08 November 1994, claims, paragraph [0002], examples, paragraph [0045] | 1-13 |
| Y | JP 2000-14388 A (ORIENTAL YEAST CO., LTD.) 18 January 2000, claims, examples, paragraph [0049] | 1-13 |
| Y | WO 2011/155358 A1 (NITTO BOSEKI CO., LTD.) 15 December 2011, claims, paragraphs [0013], [0018], examples | 1-13 |
| Y | OLIVEIRA, E. B. et al. Primary structure of human C-reactive protein. Proc. Natl. Acad. Sci. USA, 1977, vol. 74, no. 8, pp. 3148-3151, p. 3148, right column, paragraph [0003] | 1-13 |

☒ Further documents are listed in the continuation of Box C.　　☒ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| | |
|---|---|
| Date of the actual completion of the international search<br>07.12.2020 | Date of mailing of the international search report<br>22.12.2020 |
| Name and mailing address of the ISA/<br>　　Japan Patent Office<br>　　3-4-3, Kasumigaseki, Chiyoda-ku,<br>　　Tokyo 100-8915, Japan | Authorized officer<br><br>Telephone No. |

Form PCT/ISA/210 (second sheet) (January 2015)

**INTERNATIONAL SEARCH REPORT**

| International application No. |
| --- |
| PCT/JP2020/039893 |

C (Continuation).    DOCUMENTS CONSIDERED TO BE RELEVANT

| Category* | Citation of document, with indication, where appropriate, of the relevant passages | Relevant to claim No. |
| --- | --- | --- |
| Y | JP 2018-535394 A (AMGEN INC.) 29 November 2018, paragraph [0113] | 1-13 |
| Y | XIA, D. Y. et al. Primary Structure of Anodonta C-reactive Protein, Chin. J. Biochem. Mol. Biol., 2000, vol. 16, no. 2, pp. 215-223, abstract | 1-13 |
| Y | JP 2006-300758 A (APRO LIFE SCIENCE INSTITUTE INC.) 02 November 2006, claims, paragraph [0015] | 1-13 |
| Y | JP 8-233816 A (WAKO PURE CHEMICAL INDUSTRIES, LTD.) 13 September 1996, examples | 10, 12 |
| Y | JP 2011-257243 A (SHINO-TEST CORP.) 22 December 2011, paragraph [0008] | 13 |

Form PCT/ISA/210 (continuation of second sheet) (January 2015)

INTERNATIONAL SEARCH REPORT

Information on patent family members

International application No.

PCT/JP2020/039893

| Patent Documents referred to in the Report | Publication Date | Patent Family | Publication Date |
|---|---|---|---|
| JP 6-311884 A | 08.11.1994 | US 5702921 A<br>claims, examples, 1. Field of the Industrial Use, column 8 last paragraph<br>EP 622460 A2 | |
| JP 2000-14388 A | 18.01.2000 | (Family: none) | |
| WO 2011/155358 A1 | 15.12.2011 | US 2013/0212717 A1<br>claims, examples, paragraphs [0037], [0049]-[0052]<br>EP 2581446 A1 | |
| JP 2018-535394 A | 29.11.2018 | US 2018/0252728 A1<br>paragraph [0131]<br>WO 2017/049035 A1<br>EP 3349854 A1 | |
| JP 2006-300758 A | 02.11.2006 | (Family: none) | |
| JP 8-233816 A | 13.09.1996 | (Family: none) | |
| JP 2011-257243 A | 22.12.2011 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000014388 A

**Non-patent literature cited in the description**

- **TOSHIO TANAKA et al.** *Biochem. Biophys. Res. Commun.,* 2002, vol. 295, 163-166

- *Journal of Clinical Laboratory Medicine,* September 2002, vol. 46 (9), 973-981